# EUROPEAN PATENT APPLICATION

(11) **EP 2 476 677 A1**
(43) Date of publication of application: **18.07.2012**
(21) Application number: 10815137.4
(22) Date of filing: 07.09.2010
(51) Int. Cl.: C07D 401/12

(54) **PROCESS FOR PRODUCTION OF 4-(5-METHYLPYRIDIN-2-YLAMINO)PIPERIDINE-1-CARBOXYLIC ACID DERIVATIVES**

(30) Priority: 08.09.2009 JP 2009207408
(71) Applicant: Kyorin Pharmaceutical Co., Ltd., Tokyo 101-8311 (JP)
(72) Inventor: KAWAMURA, Koji, Shimotsuga-gun Tochigi 329-0114 (JP); MIYOSHI, Keita, Shimotsuga-gun Tochigi 329-0114 (JP); KOBAYASHI, Emi, Tokyo 101-8311 (JP)
(74) Representative: Vossius & Partner
(86) International application number: PCT/JP2010/005489
(87) International publication number: WO 2011/030537

(57) **Abstract**

A novel method for manufacturing 5-methyl-2-(piperidin-4-ylamino)pyridine is established. This method can be used as an industrial manufacturing method to produce a 4-(5-methylpyridin-2-ylamino)piperidine-1-carboxylic acid derivative represented by the general formula, 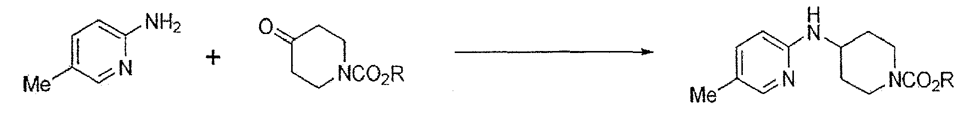 (wherein R represents a linear or branched alkyl group having 1 to 6 carbon atoms or an aralkyl group having 7 to 12 carbon atoms)
in a reaction solution having an aromatic monocyclic hydrocarbon as a reaction medium, in the presence of sodium triacetoxyborohydride as a reducing agent, or after adding sodium borohydride and acetic acid to the reaction solution in advance.

## Description

### TECHNICAL FIELD

The present invention relates to a method for manufacturing a 4-(5-methylpyridin-2-ylamino)piperidine-1-carboxylic acid derivative and 5-methyl-2-(piperidin-4-ylamino)pyridine.

### BACKGROUND ART

The N-(5-methylpyridin-2-yl)-N-(piperidinyl)-2-furancarboxamide derivative represented by the following structural formula (10),

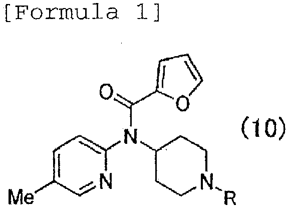

(hereinafter, referred to as "compound represented by the structural formula (10)"), is an opioid µ antagonist that has been disclosed as being effective as a prophylactic and/or a therapeutic agent for many disorders. Specifically, these disorders include side effects from µ receptor agonists, selected from constipation, nausea/emesis, and itch, as well as for diseases such as idiopathic constipation, postoperative ileus, paralytic ileus, irritable bowel syndrome, or chronic pruritus (Patent Document 1).
Further, the 5-methyl-2-(piperidin-4-ylamino)pyridine represented by the following structural formula (4),

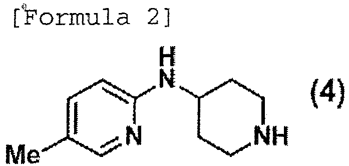

is known as a synthetic intermediate of the compound represented by the structural formula (10).
5-Methyl-2-(piperidin-4-ylamino)pyridine can be synthesized as follows, for example.
First, as shown in the below reaction pathway (a), 2-bromo-5-methylpyridine and 4-amino-1-benzylpiperidine are reacted to produce 2-(1-benzylpiperidin-4-ylamino)-5-methylpyridine. Then, under a hydrogen atmosphere, the benzyl group is removed in the presence of palladium hydroxide to produce 5-methyl-2-(piperidin-4-ylamino)pyridine.

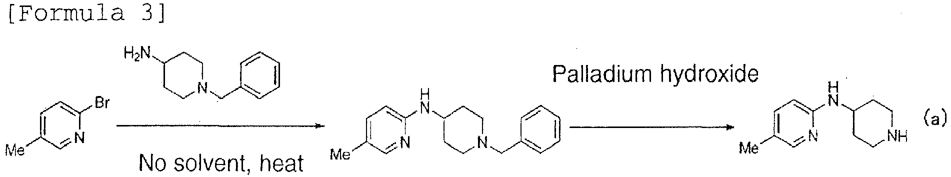

Alternatively, as shown in the below reaction pathway (b), first, 2-bromo-5-methylpyridine and ethyl 4-amino-1-piperidine carboxylate are reacted in the presence of a palladium catalyst. Next, the ethoxycarbonyl group is removed from the obtained ethyl 4-(5-methylpyridin-2-ylamino)piperidine-l-carboxylate to produce 5-methyl-2-(piperidin-4-ylamino)pyridine (Patent Document 1).

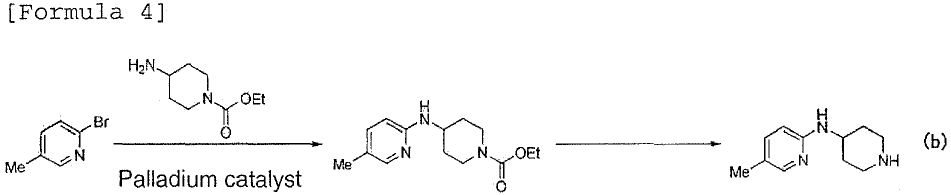

However, in the synthetic method of the reaction pathway (a), excess 4-amino-1-benzylpiperidine is required. Furthermore, the yield is low, and the reaction conditions of 180°C and 9 hours are harsh.
Moreover, in the synthetic method of the reaction pathway (b), a palladium catalyst is used. If a palladium catalyst is used for synthesis of a pharmaceutical, removal of residual palladium is known to be a problem in some cases (Non-Patent Document 1). Specifically, when an N-(5-methylpyridin-2-yl)-N-(piperidinyl)-2-furancarboxamide derivative is synthesized using a synthetic pathway including the synthetic method of the reaction pathway (b), there is a possibility that palladium will remain. Consequently, if trying to synthesize industrially, a measure for removing palladium has to be separately provided.
Further, there has also been a report of synthesizing a 2-aminopyridine derivative on the basis of a reductive amination reaction from a 2-aminopyridine derivative and a piperidone derivative shown below as reaction pathway (c). However, the yield of the 2-aminopyridine derivative obtained by this reaction pathway (c) is known to be low (Patent Document 2).

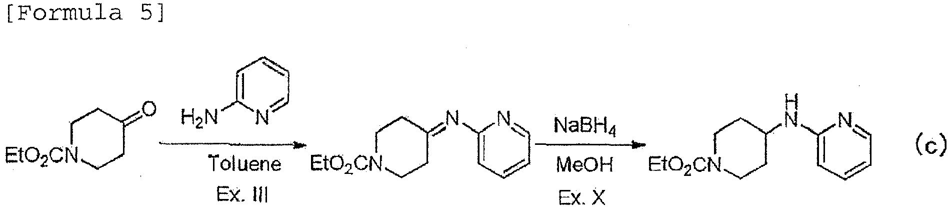

[Patent Document 1] International Publication No. WO2003/035645 pamphlet
[Patent Document 2] U.S. Pat. No. 4,126,689

[Non-Patent Document 1] Chen, C. J. Org. Chem. 2003, 68(7), 2633-38.

### SUMMARY OF THE INVENTION

### PROBLEMS TO BE SOLVED BY THE INVENTION

It is an object of the present invention to provide a manufacturing method suited to the industrial production of 5-methyl-2-(piperidin-4-ylamino)pyridine and of its precursor, 4-(5-methylpyridin-2-ylamino)piperidine-1-carboxylic acid derivative.

### MEANS FOR SOLVING THE PROBLEMS

The present inventors diligently researched a method for manufacturing a 4-(5-methylpyridin-2-ylamino)piperidine-1-carboxylic acid derivative, which is a precursor of 5-methyl-2-(piperidin-4-ylamino)pyridine, using a reductive amination reaction of a 2-aminopyridine derivative. As a result, the present inventors discovered a manufacturing method that can be utilized in an industrial manufacturing method, thereby completely the present invention.

Specifically, the subject matter of present invention is as follows.
A first aspect of the present invention relates to a method for manufacturing a 4-(5-methylpyridin-2-ylamino)piperidine-1-carboxylic acid derivative, or a pharmaceutically acceptable salt thereof, represented by the following structural formula (3),

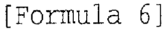

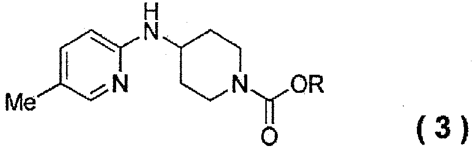

(wherein R represents a linear or branched alkyl group having 1 to 6 carbon atoms or an aralkyl group having 7 to 12 carbon atoms ),

Comprising: reacting 2-amino-5-methylpyridine represented by the following structural formula (1),

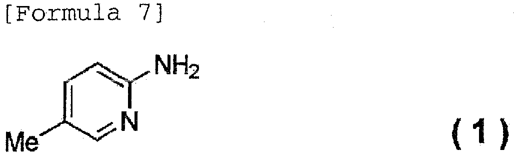

and 4-piperidone-1-carboxylic acid derivative represented by the following structural formula (2),

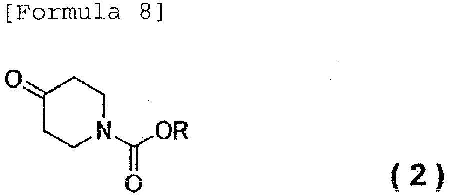

(wherein R has the same definition as the above formula (3)),

in a reaction solution having an aromatic monocyclic hydrocarbon as a reaction medium in the presence of sodium triacetoxyborohydride, or in a reaction solution into which sodium borohydride and acetic acid have been added in advance.

A second invention relates to the manufacturing method according to the first invention, wherein the aromatic monocyclic hydrocarbon is toluene.
A third invention relates to the manufacturing method according to the first invention, wherein the 2-amino-5-methylpyridine and the 4-piperidone-1-carboxylic acid derivative are reacted in the reaction solution after sodium borohydride and acetic acid have been added in advance to the reaction solution.
A fourth invention relates to the manufacturing method according to any one of the first to third inventions, wherein a reaction temperature is 50 to 70°C.
A fifth invention relates to the manufacturing method according to any one of the first to fourth inventions, wherein acetic acid is added to a reaction solution in which the 2-amino-5-methylpyridine and sodium borohydride are present, and then the 4-piperidone-1-carboxylic acid derivative is added to the reaction solution.
A sixth invention relates to themanufacturingmethodaccording to the fifth invention, wherein the reaction solution in which the 2-amino-5-methylpyridine and sodium borohydride are present is heated to a temperature of 40 to 60°C and stirred for 0.5 to 2 hours, then the reaction solution is cooled to a temperature of 0 to 20°C and then acetic acid is added, and the 4-piperidone-1-carboxylic acid derivative is added to the reaction solution.
A seventh invention relates to a method for manufacturing 5-methyl-2-(piperidin-4-ylamino)pyridine, or a pharmaceutically acceptable salt thereof, represented by the following structural formula (4),

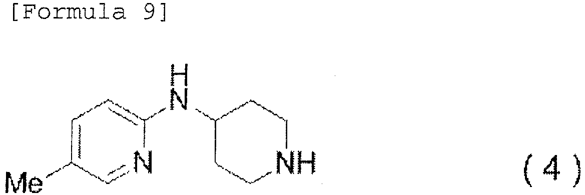

comprising: hydrolyzing the 4-(5-methylpyridin-2-ylamino)piperidine-1-carboxylic acid derivative obtained by the manufacturing method according to any one of the first to sixth inventions with an acid or a base.
An eighth invention relates to the manufacturing method according to the seventh invention, hydrolyzing the 4-(5-methylpyridin-2-ylamino)piperidine-1-carboxylic acid derivative while heating to reflux in a hydrogen bromide-acetic acid solution.

### ADVANTAGES OF THE INVENTION

According to the present invention, a 4-(5-methylpyridin-2-ylamino)piperidine-1-carboxylic acid derivative can be obtained in a good yield. Further, according to the present invention, a method for manufacturing a 4-(5-methylpyridin-2-ylamino)piperidine-1-carboxylic acid derivative can be provided which has mild reaction conditions and which is free from the problem of residual palladium. Specifically, the manufacturing method according to the present invention is useful as an industrial manufacturing method.

### EMBODIMENTS OF THE INVENTION

The term "halogen atom" in the present specification means a fluorine atom, a chlorine atom, a bromine atom, or an iodine atom.
Examples of a linear alkyl group having 1 to 6 carbon atoms include a methyl group, an ethyl group, a propyl group, an isopropyl group, a butyl group, a pentyl group, and a hexyl group.
Examples of a branched alkyl group having 1 to 6 carbon atoms include an isopropyl group, an isobutyl group, a sec-butyl group, and a t-butyl group.
Examples of a linear alkyloxy group having 1 to 6 carbon atoms include a methoxy group, an ethoxy group, a propoxy group, a butyloxy group, a pentyloxy group, and a hexyloxy group.
Examples of a branched alkyloxy group having 1 to 6 carbon atoms include an isopropoxy group, an isobutyloxy group, a sec-butyloxy group, and a t-butyloxy group.
Examples of an aralkyl group having 7 to 10 carbon atoms include a benzyl group, a phenethyl group, and a 1-phenylethyl group. One to three of the hydrogen atoms of this aralkyl groupmaybe substituted with a halogen atom, a linear or branched alkyloxy group having 1 to 6 carbon atoms, or a linear or branched alkyl group having 1 to 6 carbon atoms.

1 The amount of 4-piperidone-1-carboxylic acid derivative used in the synthetic reaction of the 4-(5-methylpyridin-2-ylamino)piperidine-1-carboxylic acid derivative is preferably from 0.7 moles to 10 moles based on 1 mole of 2-amino-5-methylpyridine, and more preferably from 1 mole to 2 moles.

A commercially-available product can be used for the 4-piperidone-1-carboxylic acid derivative and 2-amino-5-methylpyridine.
By including a step of reacting these, a 4-(5-methylpyridin-2-ylamino)piperidine-1-carboxylic acid derivative or pharmaceutically acceptable salt thereof can be obtained. In the present embodiment, examples of a pharmaceutically acceptable salt include inorganic salts of hydrochloric acid, hydrobromic acid, sulfuric acid and the like, and organic salts of acetic acid, fumaric acid, maleic acid, oxalic acid, citric acid, methanesulfonic acid, and tosylic acid.

In the synthetic reaction of the 4-(5-methylpyridin-2-ylamino)piperidine-1-carboxylic acid derivative, sodium triacetoxyborohydride is used as a reducing agent. The amount of sodium triacetoxyborohydride is preferably from 0.8 moles to 10 moles based on 1 mole of 2-amino-5-methylpyridine, and more preferably from 1 mole to 2 moles.
Further, as described below, in the present embodiment, the sodium triacetoxyborohydride may be prepared from sodium borohydride and acetic acid in the aromatic monocyclic hydrocarbon in which the synthetic reaction occurs. In this case, the amount of sodium borohydride is preferably from 0.8 moles to 10 moles based on 1 mole of 2--amino-5-methylpyridine, and more preferably from 1 mole to 2 moles. In addition, the amount of acetic acid is preferably from 2.4 moles to 3 moles based on 1 mole of 2-amino-5-methylpyridine, and more preferably from 3 moles to 6 moles.

In the synthetic reaction of the 4-(5-methylpyridin-2-ylamino)piperidine-1-carboxylic acid derivative, the reductive amination reaction is dramatically promoted if the reaction temperature is increased. On the other hand, if the reaction temperature is too high, the reduction reaction of the 4-piperidone-1-carboxylic acid derivative proceeds preferentially to the reductive amination reaction, so that the yield can decrease. Therefore, for a good yield and to shorten the reaction time, the reaction temperature may be preferably set in the range from 0°C to the reflux temperature of the solvent. More preferably, the reaction temperature is set in the range of from 25°C to 80°C. To obtain a high-purity product, it is even more preferred to set the reaction temperature in the range of from 50°C to 70°C.

In the synthetic reaction of the 4-(5-methylpyridin-2-ylamino)piperidine-1-carboxylic acid derivative, the reaction time can preferably from 10 minutes to 120 hours. More preferably, the reaction time can be from 60 minutes to 5 hours.

Examples of the aromatic monocyclic hydrocarbon used as the reaction medium in the synthetic reaction of the 4-(5-methylpyridin-2-ylamino)piperidine-1-carboxylic acid derivative include benzene, toluene, xylene, o-xylene, m-xylene, andp-xylene. Preferably, toluene is used as the aromatic monocyclic hydrocarbon.
The amount of aromatic monocyclic hydrocarbon used as the solvent is preferably from 0.5 L to 30 L based on 1 kg of 2-amino-5-methylpyridine, and more preferably from 3 L to 15 L.
In the present specification, the term "reactionmedium" refers to a substance which exists to dissolve or disperse the starting materials and the products in the medium during the reaction. In the present embodiment, the aromatic monocyclic hydrocarbon, which is the reaction medium, is also referred to below as "solvent" or "reaction solvent".
Further, in the present specification, the term "reaction solution" is a concept indicating a state in which the starting materials, the products, or other substances such as the reducing agent, are dissolved or dispersed in the reaction medium.

Further, in the present embodiment, it is preferred to add sodiumborohydride and acetic acid to the reaction solution in advance, and then react 2-amino-5-methylpyridine and the 4-piperidone-1-carboxylic acid derivative in the reaction solution.
In other words, it is preferred to prepare sodium triacetoxyborohydride from sodium borohydride and acetic acid in the reaction solution, which has an aromatic monocyclic hydrocarbon as a reaction medium.
Consequently, the yield and purity of the product obtained by a reductive amination reaction can be increased.

In a preferred embodiment, when carrying out the reductive amination reaction using sodium borohydride and acetic acid, the acetic acid is added to a reaction solution in which an aromatic monocyclic hydrocarbon is used as the solvent and 2-amino-5-methylpyridine and sodium borohydride are present. Then, the 4-piperidone-1-carboxylic acid derivative is added to this reaction solution. Carrying out the reaction in the above order is preferred from a safety perspective, as it enables severe foaming to be suppressed.
In addition, during this process, since the occurrence of severe foaming and the occurrence of byproducts can be reliably suppressed, it is preferred to stir while heating the reaction solution in which 2-amino-5-methylpyridine and sodium borohydride are present, then cool the reaction solution, and add acetic acid. At this stage, by heating the reaction solution in which 2-amino-5-methylpyridine and sodium borohydride are present to 40 to 60°C, the foaming and heat generation which occur during the step of adding the acetic acid to the reaction solution are suppressed. Further, by cooling the reaction solution to 0 to 20°C and then adding the acetic acid, byproducts and severe foaming are suppressed.
For example, as a preferred embodiment, first, the reaction solution in which 2-amino-5-methylpyridine and sodium borohydride are present is heated to a temperature of 40 to 60°C, and stirred for 0.5 to 3 hours. Then, the reaction solution is cooled to a temperature of 0 to 20°C and the acetic acid is added. Next, the 4-piperidone-1-carboxylic acid derivative is added to the reaction solution, and reacted with 2-amino-5-methylpyridine. As a more preferred embodiment, the reaction solution in which 2-amino-5-methylpyridine and sodium borohydride are present is heated to a temperature of 45 to 60°C, and stirred for 1 to 2 hours. Then, the reaction solution is cooled to a temperature of 0 to 10°C and the acetic acid is added. Next, the 4-piperidone-1-carboxylic acid derivative is added to the reaction solution, and reacted with 2-amino-5-methylpyridine.

The 4-(5-methylpyridin-2-ylamino)piperidine-1-carboxylic acid derivative obtained by the reductive amination reaction of the present embodiment can be converted into 5-methyl-2-(piperidin-4-ylamino)pyridine or a pharmaceutically acceptable salt thereof by a step including a hydrolysis reaction, which is a known deprotection condition, by an acid or a base. For example, the 4-(5-methylpyridin-2-ylamino)piperidine-1-carboxylic acid derivative can be converted into 5-methyl-2-(piperidin-4-ylamino)pyridine dihydrobromide by including a step of heating to reflux in a hydrogen bromide-acetic acid solution.
In the present embodiment, examples of a pharmaceutically acceptable salt include inorganic salts of hydrochloric acid, hydrobromic acid, sulfuric acid and the like, and organic salts of acetic acid, fumaric acid, maleic acid, oxalic acid, citric acid, methanesulfonic acid, and tosylic acid.

According to themanufacturingmethodof the present embodiment, yield can be increased compared with the conventional art. Further, compared with the conventional method, the reaction conditions are mild, and there are also no problems with residual palladium. Therefore, the manufacturing method according to the present embodiment is useful as an industrial manufacturing method. Examples

The present invention is described in more detail based on the following examples. However, the present invention is not limited to these examples.
In the examples, a purity measurement based on HPLC (high performance liquid chromatography) was carried out under the following conditions.

Column: Diameter 4.6 mm × 250 mm (W), Wakosil-II5C18 HG, manufactured by Wako
Detector: Ultraviolet absorption photometer (measurement wavelength: 240 nm)
Column temperature: Constant temperature near 40°C
Mobile phase: 2.72 g of potassium dihydrogenphosphate was dissolved in 550 mL of water. Phosphoric acid was added to adjust the pH to 2.5, and then 450 mL of acetonitrile and 1.44 g of sodium lauryl sulfate were mixed into the solution.
Flow rate: 1.0 mL/min

Although there are not many reports concerning reaction examples of a 2-aminopyridine derivative and a cyclohexanone derivative or a piperidone derivative, reaction examples in a chlorine-based solvent (methylene chloride or chloroform) are known (Japanese Translation of International Patent Application No. 2008-538775 and International Publication W02008/089201). Therefore, first, as a comparative example, a reaction in a chlorine-based solvent was investigated. Further, a reaction in an ether-based solvent was also investigated.

### <Comparative Example 1> Investigation of Reaction Solvent

A synthetic reaction was carried out based on the reaction formulae (d) and (e) shown below.

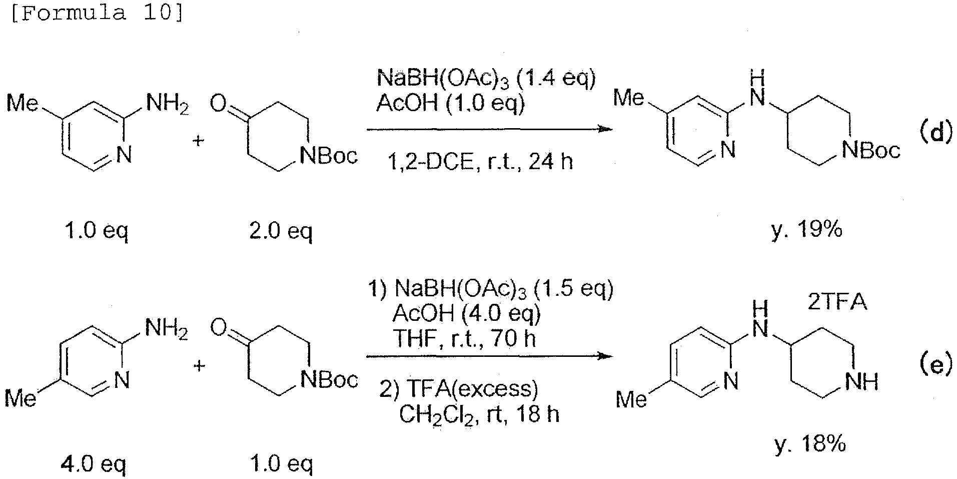

Using 2-amino-4-methylpyridine or 2-amino-5-methylpyridine and t-butyl-4-oxopiperidine-1-carboxylate, the yield in the synthesis of a 2-(piperidin-4-ylamino)methylpyridine derivative based on a reductive amination reaction was investigated. As a result, the yield in the case shown by the reaction formula (e) in the ether-based solvent (tetrahydrofuran; THF) was 18%. Further, the yield in the case shown by the reaction formula (d) in the chlorine-basedsolvent (1,2-dichloroethane; 1,2-DCE) was 19%. Thus, the yield in either case in the comparative example was low.

### Example 1

### (Use of sodium triacetoxyborohydride as a reducing agent)

### Step 1: Manufacture of ethyl

### 4-(5-methylpyridin-2-ylamino)piperidine-1-carboxylate

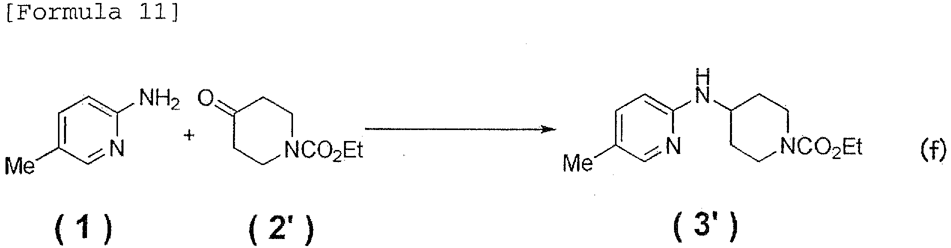

Sodium triacetoxyborohydride (215.6 g, 1.02 mol) and toluene (100 mL) were charged into a solution of 2-amino-5-methylpyridine (100 g, 0.925 mol) represented by the structural formula (1) in toluene (500 mL) to prepare a reaction solution. A solution of ethyl 4-piperidone-1-carboxylate (238 g, 1.39 mol), represented by the structural formula (2') in the reaction formula (f), in toluene (150 mL) was added dropwise at 50°C to the reaction solution, and the resultant mixture was stirred for 4 hours at 60°C.
A commercially-available product can be used for 2-amino-5-methylpyridine and ethyl 4-piperidone-1-carboxylate. Acquisition of these compounds was carried out in the same way in the below-described Example 2 as well.
The reaction solution was cooled to near room temperature, and then water (800 mL) was added. The resultant mixture was stirred for 30 minutes, then concentrated hydrochloric acid (400 mL) was added, and the mixture was stirred for 16 hours to separate the solution. The aqueous layer was washed 3 times with ethyl acetate (750 mL). The obtained aqueous layer was adjusted to a pH of 9 with 12 N sodium hydroxide aqueous solution (550 mL). The aqueous layer was extracted twice with ethyl acetate (800 mL and 400 mL), and then the combined organic layer was washed with saturated brine (250 mL). The organic layer was then dried over anhydrous magnesium sulfate and the solvent was removed by under reduced pressure, to give the title compound of ethyl 4-(5-methylpyridin-2-ylamino)piperidine-1-carboxylate (240 g) represented by the structural formula (3') in the reaction formula (f). This product was subjected to the next step without being especially purified.

### Step 2: Manufacture of

### 5-methyl-2(piperidin-4-ylamino)pyridine dihydrobromide

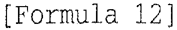

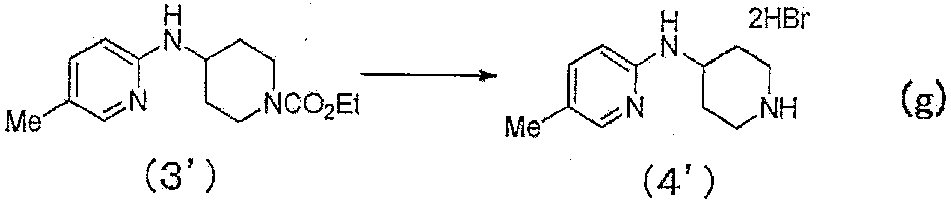

A 30 weight% hydrogen bromide in acetic acid solution (998 g, 3.70 mol) and water (66.6 mL) were charged into a solution of ethyl 4-(5-methylpyridin-2-ylamino)piperidine-1-carboxylate (239 g, ca. 0.925 mol), represented by the structural formula (3') obtained in step 1, in acetic acid (320 mL). The resultant mixture was then stirred for 4 hours under reflux condition. The reaction solution was cooled to room temperature, then charged with methanol (640 mL) and ethyl acetate (3.2 L), and stirred for 12.5 hours at room temperature. The precipitated crystals were filtered, and washed with ethyl acetate (320 mL), to givecrude 5-methyl-2-(piperidin-4-ylamino)pyridine dihydrobromide (wet weight 248 g, hereinafter referred to as crude title compound (4')) represented by the structural formula (4') in the reaction formula (g).

A solution of the crude title compound (4') (248 g) in ethanol (2.4 L) was stirred for 4 hours under reflux condition. Next, this ethanol solution was stirred for 16 hours at room temperature. The ethanol solution was then stirred for 3 hours under ice cooling. The precipitated crystals were filtered, washed with ethanol (320 mL), and dried under reduced pressure, to give the title compound of 5-methyl-2-(piperidin-4-ylamino)pyridine dihydrobromide (142 g, overallyieldviatwosteps: 43.5%, HPLC purity: > 99%) represented by the structural formula (4').

### Example 2

### (Use of sodium triacetoxyborohydride as a reducing agent, prepared from sodium borohydride and acetic acid)

### Step 1: Manufacture of ethyl

### 4-(5-methylpyridin-2-ylamino)piperidine-1-carboxylate

2-Amino-5-methylpyridine (100 g, 0.925 mol) represented by the structural formula (1) was charged into a suspension of sodium borohydride (38.5 g, 1.02 mol) in toluene (600 mL) to prepare a reaction solution. This reaction solution was stirred for 1 hour at 52°C. This reaction solution was cooled under ice cooling, and then acetic acid (183 g, 3.05 mol) was added dropwise to the reaction solution at 4 to 6°C. The reaction solution was stirred at the same temperature for 15 hours, and then stirred for 1 hour at 50°C.

A solution of ethyl 4-piperidone-1-carboxylate (238 g, 1.39 mol) represented by the structural formula (2') in toluene (150 mL) was added dropwise to the reaction solution, and the resultant mixture was stirred for 4 hours at 60°C. The reaction solution was cooled to near room temperature, and then water (800 mL) was added. The resul tant mixture was stirred for 30 minutes, then concentrated hydrochloric acid (400 mL) was added, and the mixture was stirred for 16 hours to separate the solution. The aqueous layer was washed with ethyl acetate (750 mL). The obtained aqueous layer (1,650 mL) was adjusted to a pH of 9 with 12 N sodium hydroxide aqueous solution (540 mL). The aqueous layer was extracted twice with ethyl acetate (800 mL and 400 mL), and the combined organic layer was then washed twice with water and saturated brine (300 mL + 100 mL) and once with saturated brine (300 mL). The organic layer was then dried over anhydrous magnesium sulfate, and the solvent was removed under reduced pressure, to give the title compound of ethyl 4-(5-methylpyridin-2-ylamino)piperidine-1-carboxylate (249 g) represented by the structural formula (3'). This product was subjected to the next step without being especially purified.

### Step 2: Manufacture of

### 5-methyl-2-(piperidin-4-ylamino)pyridine dihydrobromide

A 30 weight% hydrogen bromide-acetic acid solution (998 g, 3.70 mol) and water (66.6 mL) were charged into a solution of ethyl 4-(5-methylpyridin-2-ylamino)piperidine-1-carboxylate (249 g, ca. 0.925 mol), represented by the structural formula (3'), in acetic acid (320 mL). The resultant mixture was then stirred for 4 hours at 120°C. The reaction solution was cooled to room temperature, then charged with methanol (640 mL) and ethyl acetate (3.2 L), and stirred for 16 hours at room temperature. The precipitated crystals were filtered, and washed with ethyl acetate (320 mL), to give crude 5-methyl-2-(piperidin-4-ylamino)pyridine dihydrobromide (wet weight 375 g, hereinafter referred to as crude title compound (4')) represented by the structural formula (4').

A solution of the crude title compound (4') (375 g) in ethanol (2.4 L) was stirred for 4 hoursunder reflux condition, then stirred for 16 hours at room temperature, and stirred for 3 hours under ice cooling. The precipitated crystals werefiltered, washed with ethanol (320 mL), and dried under reduced pressure, to give the title compound of 5-methyl-2-(piperidin-4-ylamino)pyridine dihydrobromide (203 g, overall yield via two steps: 62.1%, HPLC purity: > 99%) represented by the structural formula (4').
¹H-NMR (DMSO-d6) δ 1.75-1.85 (m, 2H), 2.11-2.14(m, 2H), 2.20 (s, 3H), 3.01-3.06 (m, 2H), 3.37-3.42 (m, 2H), 4.04-4.09 (m, pH), 7.13 (d, 1H, J = 9.3 Hz), 7.77 (s, 1H), 7.81 (d, 1H, J = 9.3 Hz), 8.73 (br, 1H), 8.85 (br, 1H).

From the above comparative examples and working examples, it can be seen that when a reductive amination reaction is carried out with toluene, which is an example of an aromatic monocyclic hydrocarbon, as a reaction solvent, the yield of ethyl 4-(5-methylpyridin-2-ylamino)piperidine-1-carboxylate is dramatically higher than when the reaction is carried out with tetrahydrofuran or 1,2-dichloroethane.

Further, according to the present embodiment, even when 100 g of the rawmaterial compound of 2-amino-5-methylpyridine was used, ethyl 4-(5-methylpyridin-2-ylamino)piperidine-1-carboxylate was obtained safely and in a good yield. In addition, the 5-methyl-2-(piperidin-4-ylamino)pyridine salt derived from this ethyl 4-(5-methylpyridin-2-ylamino)piperidine-1-carboxylate had a high purity. Especially, in Example 2, the reaction could be carried out under even milder conditions. Moreover, the yield was even higher than in Example 1. Therefore, the manufacturing method according to the present embodiment can be utilized even for industrial-scale production.

### INDUSTRIAL APPLICABILITY

Anovel manufacturingmethodwas established for manufacturing a 4-(5-methylpyridin-2-ylamino)piperidine-1-carboxylic acid derivative from 2-amino-5-methylpyridine and a 4-piperidone-1-carboxylic acid derivative. This manufacturing method is suitable as an industrial manufacturing method, as it canobtaina4-(5-methylpyridin-2-ylamino)piperidine-1-carboxylic acid derivative in a good yield without using a heavy metal reagent such as palladium, under mild reaction conditions. Further, 5-methyl-2-(piperidin-4-ylamino)pyridine derived from the 4-(5-methylpyridin-2-ylamino)piperidine-1-carboxylic acid derivative has a high purity. Using this 5-methyl-2-(piperidin-4-ylamino)pyridine as a raw material, an N-(5-methylpyridin-2-yl)-N-(4-piperidinyl)-2-furancarboxamide derivative, which is an opioid µ antagonist, can be produced.

## Claims

1. A method for manufacturing a 4-(5-methylpyridin-2-ylamino)piperidine-1-carboxylic acid derivative, or a pharmaceutically acceptable salt thereof, represented by the following structural formula (3), 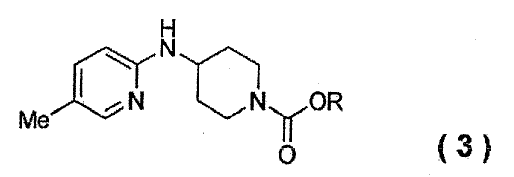 (wherein R represents a linear or branched alkyl group having 1 to 6 carbon atoms or an aralkyl group having 7 to 12 carbon atoms), comprising:
reacting 2-amino-5-methylpyridine represented by the following structural formula (1), 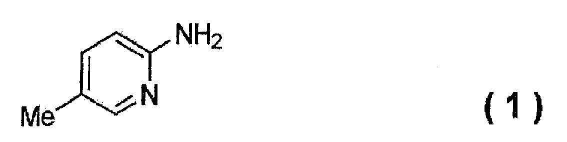 and 4-piperidone-1-carboxylic acid derivative represented by the following structural formula (2), 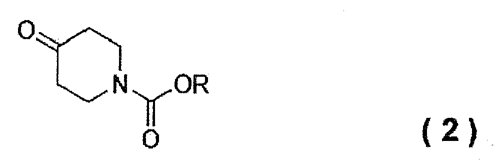 (wherein R has the same definition as the above formula (3)), in a reaction solution having an aromatic monocyclic hydrocarbon as a reaction medium in the presence of sodium triacetoxyborohydride, or in a reaction solution into which sodium borohydride and acetic acid have been added in advance.

2. The manufacturing method according to claim 1, wherein the aromatic monocyclic hydrocarbon is toluene.

3. The manufacturing method according to claim 1, wherein the 2-amino-5-methylpyridine and the 4-piperidone-1-carboxylic acid derivative are reacted in the reaction solution after sodium borohydride and acetic acid have been added in advance to the reaction solution.

4. The manufacturing method according to any one of claims 1 to 3, wherein a reaction temperature is 50 to 70°C.

5. The manufacturing method according to any one of claims 1 to 4, wherein acetic acid is added to a reaction solution in which the 2-amino-5-methylpyridine and sodium borohydride are present, and
then the 4-piperidone-1-carboxylic acid derivative is added to the reaction solution.

6. The manufacturing method according to claim 5, wherein the reaction solution in which the 2-amino-5-methylpyridine and sodium borohydride are present is heated to a temperature of 40 to 60°C and stirred for 0.5 to 3 hours,
then the reaction solution is cooled to a temperature of 0 to 20°C and then acetic acid is added, and
the 4-piperidone-1-carboxylic acid derivative is added to the reaction solution.

7. A method for manufacturing 5-methyl-2-(piperidin-4-ylamino)pyridine, or a pharmaceutically acceptable salt thereof, represented by the following structural formula (4), 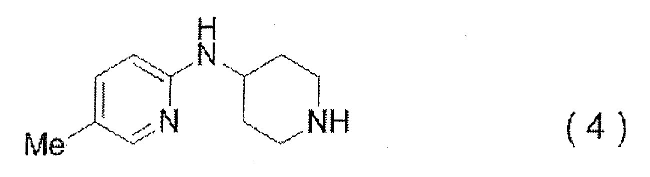 Comprising:
hydrolyzing the 4-(5-methylpyridin-2-ylamino)piperidine-1-carboxylic acid derivative obtained by the manufacturing method according to any one of claims 1 to 6 with an acid or a base.

8. The manufacturing method according to claim 7, hydrolyzing the 4-(5-methylpyridin-2-ylamino)piperidine-1-carboxylic acid derivative while heating to reflux in a hydrogen bromide-acetic acid solution.
